# EUROPEAN PATENT SPECIFICATION

(11) **EP 3 273 894 B1**
(45) Date of publication and mention of the grant of the patent: **15.03.2023**
(21) Application number: 16716995.2
(22) Date of filing: 25.03.2016
(51) Int. Cl.: A61B 34/20

(54) **ANATOMIC REGISTRATION PROBES, SYSTEMS AND METHODS**
ANATOMISCHE REGISTRIERUNGSSONDEN, SYSTEME UND VERFAHREN
SONDES D'ALIGNEMENT ANATOMIQUE, SYSTÈMES ET PROCÉDÉS

(30) Priority: 26.03.2015 US 201562138447 P
(43) Date of publication of application: 31.01.2018
(73) Proprietor: Biomet Manufacturing, LLC, Warsaw, IN 46582 (US)
(72) Inventor: BEREND, Keith R., Columbus, OH 43215 (US); WHITE, John, Winona Lake, IN 46590 (US)
(74) Representative: Mays, Julie
(86) International application number: PCT/US2016/024240
(87) International publication number: WO 2016/154548

(56) References cited:
- EP-A1- 2 298 215
- WO-A1-2014/176207
- US-A1- 2011 082 383
- US-A1- 2012 020 547
- US-A1- 2014 187 955
- US-A1- 2014 314 297

## Description

### FIELD

The present disclosure relates to anatomic registration probes and associated systems and methods.

### BACKGROUND

This section provides background information related to the present disclosure, which is not necessarily prior art.

Surgical navigation is commonly used for various types of orthopedic surgeries. Surgical navigation systems typically require registration to the patient's anatomy. One common method of anatomical registration employs a stylus having a locator disposed on one end in conjunction with a detection unit in order to sequentially record anatomical landmarks. Because human anatomy can be geometrically complex, it is often necessary for a stylus to register hundreds or thousands of points in order to adequately define relevant patient anatomy. US2014/187955 discloses systems and methods of registration using an ultrasound probe. US2011/082383 shows systems and methods for discriminating and locating tissues within a body and US2014/314297 shows a registering method, a position detecting system and the corresponding scanning instrument.

### SUMMARY

This section provides a general summary of the disclosure, and is not a comprehensive disclosure of its full scope or all of its features.

The present inventors have recognized that the process of sequentially recording individual anatomical points is arduous and time consuming. Devices and systems for facilitating more efficient anatomical registration would therefore be desirable. Accordingly, the present teachings provide for a system for efficient anatomical registration. The present invention provides a system for anatomical registration as defined in claim 1. Preferred embodiments are defined by the dependent claims.

Aspects, embodiments, examples and methods disclosed hereinafter which do not fall within the scope of the appended claims do not form part of the present invention.

The present teachings also provide for a method for efficient anatomical registration. The method does not form part of the present invention and comprises: activating a data collection unit for a selected time period; placing a dynamic engagement tip of a registration probe into contact with a selected anatomical area, wherein the registration probe has a distal portion, an intermediate portion, a proximal portion, and a longitudinal axis defined by the intermediate portion, wherein the dynamic engagement tip is operably coupled to a distal end of the distal portion, and wherein the dynamic engagement tip is rotatable with respect to the registration probe; moving the registration probe relative to the selected anatomical area via rotation of the dynamic engagement tip; recording physical space data corresponding to individual surface points at a data collection rate during the selected time period; determining at least one of a plurality of point-based registrations and distance between individual surface points based on the collected physical space data..

The present teachings further provide for another device for efficient anatomical registration. This device does not form part of the present invention and can comprise a registration probe body and a functional engagement tip. The registration probe can have a distal portion, intermediate portion, a proximal portion, and a longitudinal axis defined by the intermediate portion. The functional engagement tip can be coupled to a distal end of the distal portion. The functional engagement tip can further comprise a contact surface geometry that can be complementary to a selected anatomical area. The device can also comprise a locator coupled to the proximal portion. In operation, the functional engagement tip of the registration probe can facilitate registration of at least one of the size, location, and orientation of the selected anatomical area.

The present teachings further provide for another method for efficient anatomical registration which does not form part of the present invention, said method comprising: activating a data collection unit; placing a functional engagement tip of a registration probe into contact with a selected anatomical area, wherein the registration probe has a distal portion, an intermediate portion, a proximal portion, and a longitudinal axis defined by the intermediate portion, wherein the functional engagement tip is coupled to a distal end of the distal portion, and wherein the functional engagement tip comprises a contact surface geometry that is complementary to a selected anatomical area; and recording at least one of the size, location and orientation of the functional engagement tip to facilitate registration of at least one of the size, location, and orientation of the selected anatomical area.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Figure 1 illustrates an anatomical registration system;
Figure 2 illustrates one aspect of a registration probe;
Figure 3 illustrates another aspect of a registration probe;
Figure 4 illustrates another aspect of a registration probe;
Figure 5 illustrates another aspect of a registration probe;
Figure 6 illustrates another aspect of a registration probe;
Figure 7 illustrates another aspect of a registration probe; and
Figure 8 is a flow chart illustrating an exemplary process of anatomical registration.

Corresponding reference numerals indicate corresponding parts throughout the several views of the drawings.

### DETAILED DESCRIPTION

Example embodiments will now be described more fully with reference to the accompanying drawings.

The present teachings provide for various systems, devices, and methods for facilitating efficient anatomical registration in surgical navigation. Such systems, devices, and methods can provide for relatively rapid collection of physical space data corresponding to individual surface points during a dynamic data collection mode of a detection unit. Additionally or alternatively, such systems, devices, and methods can provide for collection of a plurality of data at a single point collection mode of a detection unit.

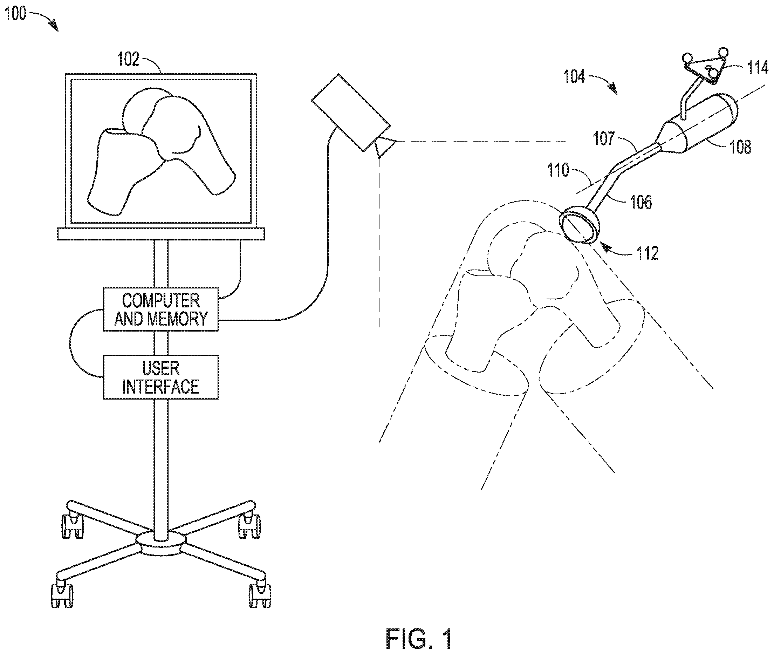

With initial reference to Figure 1, a system 100 comprises a data collection unit 102 and a registration probe 104. The data collection unit 102and registration probe of the present teachings can be used in combination for anatomical registration prior to and during a surgical procedure.

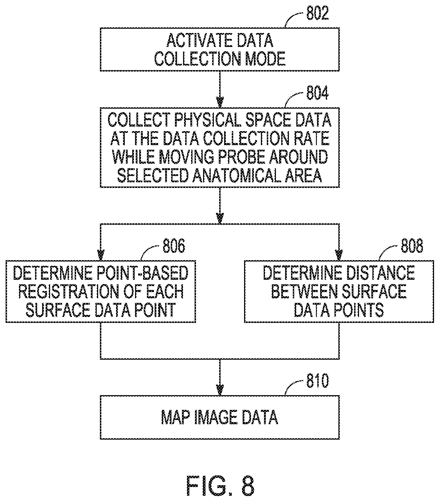

In one aspect, the data collection unit 102 can include, for example and without limitation, a computer assisted surgery system or a surgical navigation system. The data collection unit 102 can comprise, for example and without limitation, a camera, a vision system, and the like. The data collection unit 102 can have a data collection mode that can be selectively activated by a user via an activating mechanism. The data collection unit 102 can have a predefined data collection period for the data collection mode or can be selectively deactivated by a user via a termination mechanism. The data collection rate can be between about 1Hz to about 60Hz. As shown in Figure 8, in operation and upon activation of the data collection mode (step 802), the data collection unit 102 can collect physical space data corresponding to each of a plurality of surface points on the selected anatomical area (step 804). The physical space data corresponding to each surface data point can be used to determine point-based registration (step 806). The physical space data corresponding to at least two sequential surface points can be used to determine the distance of travel of the registration probe 104 across the selected anatomical area and, thus, the distance between surface points (step 808). Next, the data collection unit maps image data describing the selected anatomical area using at least the point-based registration of each of the plurality of surface points (step 810).

Registration probes 104 according to the present teachings can have a distal portion 106, an intermediate portion 107, a proximal portion 108, and a longitudinal axis 110 defined by the intermediate portion 107. The registration probe can have an overall length of between about 100mm to about 400mm along its longitudinal axis 110. Furthermore, at least a portion of one of the distal portion 106 and the proximal portion 108 can be straight, curved, bent or offset from the registration probe longitudinal axis 110. Accordingly, the registration probe can be adapted to suit a wide variety of anatomical features as well as sizes and shapes of anatomical features.

In one aspect, the registration probe 104 can further comprise an engagement tip 112 operably coupled to the distal end of the distal portion 106. The engagement tip 112can comprise a dynamic engagement tip 112 and the dynamic engagement tip can further comprise a roller. The registration probe can also comprise a locator 114 coupled to the proximal portion 108. The dynamic engagement tip 112 can be rotatable with respect to the registration probe 104. In operation, the dynamic engagement tip 112of the registration probe 104 can be placed in continuous and dynamic contact with and relative to a selected anatomical area. Dynamic contact can include moving the registration probe around the surface of the anatomical area. The data collection unit 102 can collect physical space data corresponding to individual surface points at a selected data collection rate during the selected time period that the data collection unit 102 is in data collection mode.

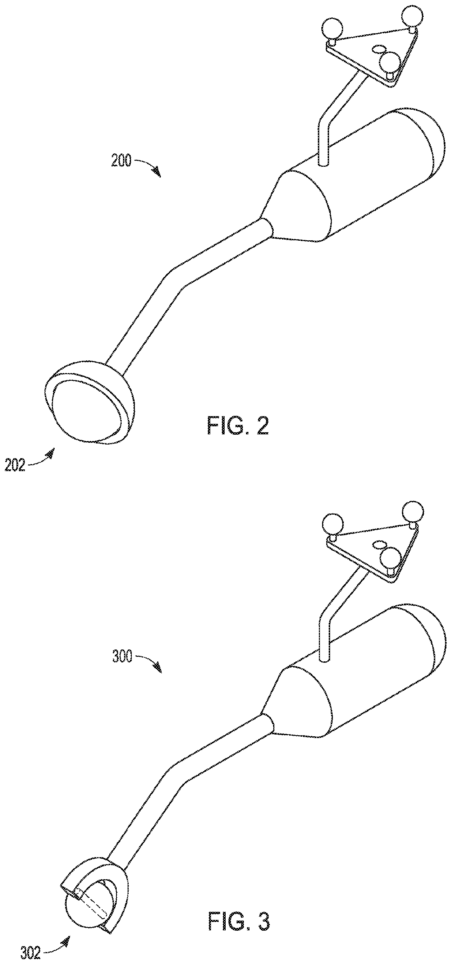

With reference to Figure 2, the dynamic engagement tip of the registration probe 200 comprises a radially unconstrained roller 202. The radially unconstrained roller 202 can move in any direction as it is rolled over the surface of the selected anatomical area. The diameter of the radially unconstrained roller 202 can be between about 5mm to about 25mm. Registration probe 200 can be used to facilitate registration of a wide variety of complex three-dimensional anatomic surfaces including their proximity to joints. Such complex three-dimensional anatomical surfaces can comprise at least portions of, for example and without limitation, the proximal and distal femur; the pelvis and acetabular region; the proximal tibia; the proximal humerus and scapula; the distal humerus and proximal radius; and the fibula, distal tibia, talus, and medial malleolus of the fibula. In operation, the data collection mode of the data collection unit 102 can be activated for a selected time period. During this time period, the radially unconstrained roller 202 can be maintained in contact with a selected anatomical area and the registration probe 200 can be moved around the selected anatomical area via the radially unconstrained roller 202 while the data collection unit 102 collects physical space data corresponding to individual surface points at the selected data collection rate. The data collection unit 102 can compensate for the shape and size of the radially unconstrained roller 202 along with the locator 114. The data collection unit 102 can also compute the distance traveled between physical space data corresponding to individual surface points.

With reference to Figure 3, the dynamic engagement tip of the registration probe 300, which does not form part of the present invention, can be a radially constrained roller 302. The roller 302 can be constrained to roll along a selected axis. The diameter of the radially constrained roller 302 can be between about 5mm to about 25mm. Registration probe 300 can be used for registering surfaces of a long bone that are aligned with the long axis of a long bone or that are transverse to the long axis of a long bone. Such anatomical surfaces can comprise at least portions of, for example and without limitation, circumferential anatomy of the proximal and distal femur, or proximal tibia, that is transverse or substantially transverse to the long axis. In operation, the data collection mode of the data collection unit 102 can be activated for a selected time period. During this time period, the radially constrained roller 302 can be maintained in contact with a selected anatomical area and the registration probe 300 can be moved along an axis relative to the selected anatomical area via rotation of the radially constrained roller 302 while the data collection unit 102 collects physical space data corresponding to individual surface points at the selected data collection rate. The data collection unit 102 can compensate for the shape and size of the radially constrained roller 302 along with the locator 114. The data collection unit 102 can also compute the distance traveled between individual surface points.

In another aspect, the data collection unit 102 can be, for example and without limitation, a computer assisted surgery system or a surgical navigation system. The data collection unit 102 can comprise, for example and without limitation, a camera, a vision system, and the like. The data collection unit 102 can have a data collection mode adapted for serial single point collection of data.

Here, the registration probe can further comprise an engagement tip 112 operably coupled to the distal end of the distal portion. The engagement tip can comprise a functional engagement tip 112 having a contact surface geometry that is complementary to a selected anatomical area. In operation, a data collection unit 102 can be activated and the functional engagement tip 112 placed into contact with a selected anatomical area. Upon placement, physical space data indicative of at least one of the size, location, and orientation of the functional engagement tip 112 can be recorded to facilitate registration of at least one of the size, location, and orientation of the selected anatomical area.

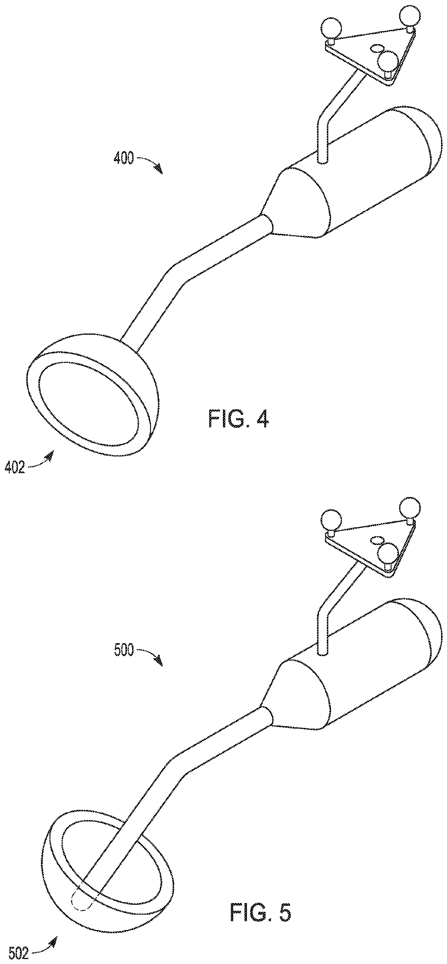

With respect to Figure 4, the functional engagement tip 112 of the registration probe 400 comprises a convex contact surface 402. A registration probe 400 having a tip comprising a convex contact surface 402 can be used for registering the size, location, and orientation of concave anatomical features such as, for example and without limitation, the acetabulum.

With respect to Figure 5, the functional engagement tip 112 of the registration probe 500 comprises a concave contact surface 502. A registration probe 500 having a tip comprising concave contact surface 502 can be used for registering the size, location, and orientation of convex anatomical features such as, for example and without limitation, the femoral head.

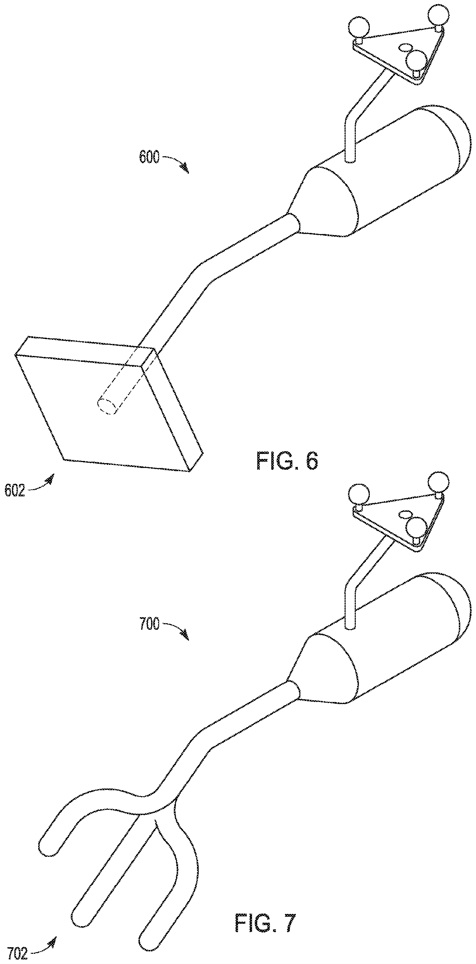

With respect to Figure 6, the functional engagement tip 112 of the registration probe 600 comprises a planar contact surface 602. A registration probe 600 having a tip comprising a planar contact surface 602 can be used for registering planar features experienced or created during the process of joint replacement such as, for example and without limitation, the flat surfaces created by proximal tibial osteotomies, distal femoral osteotomies, anterior or posterior femoral osteotomies, and femoral chamfer osteotomies. In one operational example related to a hip joint, the femoral osteotomy plane can be recorded as well as the plane of the rim of the acetabulum.

With respect to Figure 7, the functional engagement tip 112 of the registration probe 700 comprises a tip comprising an array of points 702. The tip comprising an array of points 702 can comprise at least three points. The array of points 702 can be used to register the orientation and location of a corresponding number of points on a complex anatomical surface simultaneously. The array of points 702 can be configured such that all points of the array of points 702 contact the selected anatomical surface in order to ensure accurate anatomical registration. A registration probe 700 having a tip comprising an array of points 702 can be used for registering a corresponding array of points on, for example and without limitation, the proximal femur, the distal femur, the proximal tibia, the pelvis, the acetabulum, the proximal humerous, the distal humerous, the proximal radius, and the proximal ulna. The array of points 702 can be defined with a circumscribed circle and, in a further aspect, the circumscribed circle can be between about .5 to about 3 inches in diameter.

In another aspect, the functional engagement tip 112 can be removably coupled to the distal portion 106 of the registration probe 104. Accordingly, a system comprising a registration probe 104 and a plurality of interchangeable functional engagement tips 112 is contemplated.

In yet other aspects, the data collection unit 102 can have a dynamic data collection mode as described with reference to Figures 2 and 3 and a single point collection mode as described with reference to Figures 4-7.

The foregoing description of the embodiments has been provided for purposes of illustration and description. It is not intended to be exhaustive or to limit the disclosure. Modifications and variations are possible without departing from the scope of the invention as defined by the appended claims.

## Claims

1. A system (100) for anatomical registration, comprising:
a registration probe (104), comprising:
a registration probe body having a distal portion (106), an intermediate portion (107), a proximal portion (108), and a longitudinal axis (110) defined by the intermediate portion (107);
a dynamic engagement tip (112) operably coupled to a distal end of the distal portion (106), wherein the dynamic engagement tip (112) that is rotatable with respect to the registration probe (104); and
a locator (114) coupled to the proximal portion (108); and
a data collection unit (102),
wherein, in operation, the dynamic engagement tip (112) of the registration probe (104) is placed in continuous contact with and moved around a selected anatomical area during anatomical registration, wherein the dynamic engagement tip comprises a radially unconstrained roller (202) configured to move in any direction as it is rolled over a surface of the selected anatomical area so that the registration probe is movable relative to the selected anatomical area via rotation of the radially unconstrained roller (202), wherein the data collection unit (102) is configured to collect physical space data corresponding to a plurality of surface points at a data collection rate during a selected time period of anatomical registration; and
wherein the data collection unit is configured to determine at least one of a plurality of point-based registrations and distance between individual surface points from the physical space data.

2. The system of claim 1, wherein the data collection rate is between about 1 to about 60 Hz.

3. The system of any one or combination of claims 1-2, wherein the registration probe has an overall length of between about 100mm to about 400mm along the registration probe axis.

4. The system of any one or combination of claims 1-3, wherein at least a portion of one of the distal portion and the proximal portion is straight such that it extends along the registration probe axis.

5. The system of any one or combination of claims 1-4, wherein at least a portion of one of the distal portion and the proximal portion is curved relative to the registration probe axis.

6. The system of any one or combination of claims 1-5, wherein at least a portion of one of the distal portion and the proximal portion is bent relative to the registration probe axis.

7. The system of any one or combination of claims 1-6, wherein at least a portion of one of the distal portion and the proximal portion is offset from the registration probe axis.

## Patentansprüche

1. System (100) zur anatomischen Untersuchung, umfassend:
eine Untersuchungssonde (104), umfassend:
einen Untersuchungssondenkörper, der einen distalen Abschnitt (106), einen Zwischenabschnitt (107), einen proximalen Abschnitt (108) und eine durch den Zwischenabschnitt (107) definierten Längsachse (110) aufweist;
eine dynamische Eingriffsspitze (112), die funktionsfähig mit einem distalen Ende des distalen Abschnitts (106) gekoppelt ist, wobei die dynamische Eingriffsspitze (112) in Bezug auf die Untersuchungssonde (104) drehbar ist; und
einen mit dem proximalen Abschnitt (108) verbundenen Steller (114); und
eine Datenerfassungseinheit (102),
wobei die dynamische Eingriffsspitze (112) der Untersuchungssonde (104) im Betrieb in ständigem Kontakt mit einem ausgewählten anatomischen Bereich platziert und während der anatomischen Untersuchung um diesen herum bewegt wird, wobei die dynamische Eingriffsspitze eine radial nicht eingespannte Rolle (202) umfasst, die konfiguriert ist, um sich in jede Richtung zu bewegen, wenn sie über eine Oberfläche des ausgewählten anatomischen Bereichs gerollt wird, sodass die Untersuchungssonde in Bezug auf den ausgewählten anatomischen Bereich über die Drehung der radial nicht eingespannten Rolle (202) beweglich ist,
wobei die Datenerfassungseinheit (102) konfiguriert ist, um während einer ausgewählten Zeitperiode der anatomischen Untersuchung mit einer Datenerfassungsrate physikalische Raumdaten zu erfassen, die einer Vielzahl von Oberflächenpunkten entsprechen; und
wobei die Datenerfassungseinheit so konfiguriert ist, um mindestens eines von einer Vielzahl von punktbasierenden Untersuchungen und einen Abstand zwischen einzelnen Oberflächenpunkten aus den physikalischen Raumdaten zu bestimmen.

2. System nach Anspruch 1, wobei die Datenerfassungsrate zwischen etwa 1 und etwa 60 Hz ist.

3. System nach einem oder einer Kombination der Ansprüche 1-2, wobei die Untersuchungssonde eine Gesamtlänge zwischen etwa 100 mm und etwa 400 mm entlang der Untersuchungssondenachse aufweist.

4. System nach einem der Ansprüche 1-3 oder einer Kombination davon, wobei mindestens ein Abschnitt des einen von dem distalen Abschnitt oder dem proximalen Abschnitt gerade ist, sodass er sich entlang der Untersuchungssondenachse erstreckt.

5. System nach einem oder einer Kombination der Ansprüche 1-4, wobei mindestens ein Abschnitt von einem von dem distalen Abschnitt oder dem proximalen Abschnitt in Bezug auf die Untersuchungssondenachse gekrümmt ist.

6. System nach einem oder einer Kombination der Ansprüche 1-5, wobei mindestens ein Abschnitt von einem von dem distalen Abschnitt oder dem proximalen Abschnitt in Bezug auf die Untersuchungssondenachse gebogen ist.

7. System nach einem oder einer Kombination der Ansprüche 1-6, wobei mindestens ein Abschnitt von einem von dem distalen Abschnitt oder dem proximalen Abschnitt von der Untersuchungssondenachse versetzt ist.

## Revendications

1. Système (100) permettant un alignement anatomique, comprenant :
une sonde d'alignement (104), comprenant :
un corps de sonde d'alignement comportant une partie distale (106), une partie intermédiaire (107), une partie proximale (108) et un axe longitudinal (110) défini par la partie intermédiaire (107) ;
une pointe de mise en prise dynamique (112) couplée de manière fonctionnelle à une extrémité distale de la partie distale (106), ladite pointe de mise en prise dynamique (112) étant rotative par rapport à la sonde d'alignement (104) ; et
un localisateur (114) couplé à la partie proximale (108) ; et
une unité de collecte de données (102),
en fonctionnement, ladite pointe de mise en prise dynamique (112) de la sonde d'alignement (104) étant mise en contact continu avec et déplacée autour d'une zone anatomique choisie pendant l'alignement anatomique, ladite pointe de mise en prise dynamique comprenant un rouleau radialement non contraint (202) configuré pour se déplacer dans n'importe quelle direction lorsqu'il est roulé sur une surface de la zone anatomique sélectionnée de sorte que la sonde d'alignement soit mobile par rapport à la zone anatomique choisie par rotation du rouleau radialement non contraint (202),
ladite unité de collecte de données (102) étant configurée pour collecter les données spatiales physiques correspondant à une pluralité de points de surface à une fréquence de collecte de données pendant une période de temps choisie d'alignement anatomique ; et
ladite unité de collecte de données étant configurée pour déterminer au moins l'un d'une pluralité d'alignements basés sur des points et la distance entre les points de surface individuels à partir des données spatiales physiques.

2. Système selon la revendication 1, ladite fréquence de collecte de données étant comprise entre environ 1 et environ 60 Hz.

3. Système selon l'une quelconque ou une combinaison des revendications 1 à 2, ladite sonde d'alignement présentant une longueur totale comprise entre environ 100 mm et environ 400 mm le long de l'axe de la sonde d'alignement.

4. Système selon l'une quelconque ou une combinaison des revendications 1 à 3, au moins une partie d'une partie parmi la partie distale et la partie proximale étant droite de sorte qu'elle s'étende le long de l'axe de la sonde d'alignement.

5. Système selon l'une quelconque ou une combinaison des revendications 1 à 4, au moins une partie d'une partie parmi la partie distale et la partie proximale étant incurvée par rapport à l'axe de la sonde d'alignement.

6. Système selon l'une quelconque ou une combinaison des revendications 1 à 5, au moins une partie d'une partie parmi la partie distale et la partie proximale étant courbée par rapport à l'axe de la sonde d'alignement.

7. Système selon l'une quelconque ou une combinaison des revendications 1 à 6, au moins une partie d'une partie parmi la partie distale et la partie proximale étant décalée par rapport à l'axe de la sonde d'alignement.
